# DEMANDE DE BREVET EUROPEEN

(11) **EP 0 683 161 A1**
(43) Date de publication de la demande: **22.11.1995**
(21) Numéro de dépôt: 94401087.5
(22) Date de dépôt: 17.05.1994
(51) Int. Cl.: C07D 295/08, A61K 31/445

(54) **Nouvel ester de l'acide 4-amino-5-chloro-2-méthoxybenzoique, procédé pour sa préparation et compositions pharmaceutiques le contenant**

(71) Demandeur: MIDY S.p.A., I-20137 Milano (IT)
(72) Inventeur: Langlois, Michel, F-92330 Sceaux (FR); Guzzi, Umberto, I-20100 Milano (IT); Cecchi, Roberto, I-20075 Lodi (Milano) (IT); Croci, Tiziano, I-20155 Milano (IT)
(74) Mandataire: Gillard, Marie-Louise

(57) **Abrégé**

L'invention concerne un procédé de désodorisation catalytique et de réduction de la teneur en azote des effluents de cuve à lisier.

Le procédé comporte 3 étapes :
(a) transformation des mercaptans en disulfures et de l'hydrogène sulfuré en sulfures.
(b) Post combustion catalytique des composés organiques volatils
(c) Réduction catalytique des oxydes d'azote.

## Description

La présente invention concerne un nouveau composé de formule (I) 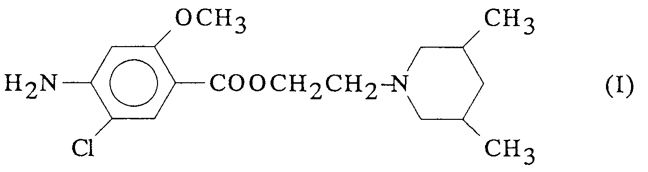
ainsi que ses sels pharmaceutiquement acceptables et ses sels d'ammonium quaternaires, le procédé pour sa préparation, les compositions pharmaceutiques le contenant et son application thérapeutique en tant qu'antagoniste sélectif du récepteur 5-HT₄.

Les amides de l'acide 4-amino-5-chloro-2-méthoxy benzoïque sont connus depuis longtemps; le métoclopramide et le cisapride sont deux exemples de cette classe de composés qui ont trouvé un emploi en thérapeutique en tant que gastroprocinétiques; ces composés ne sont pas sélectifs et possèdent, entre autre, des propriétés antagonistes du récepteur 5-HT₃ .

Plus particulièrement, DE-1233877 décrit des dérivés des benzamides, notamment le composé de formule (II)

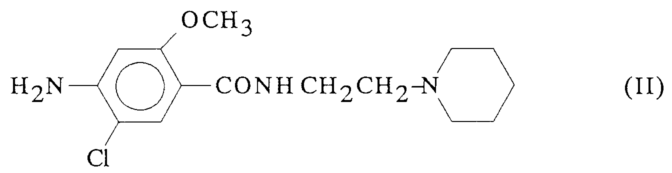

BE-901274, 900425, 897117 décrivent des composés similaires, à activité 5-HT₃ antagoniste parmi lesquels les esters de formule 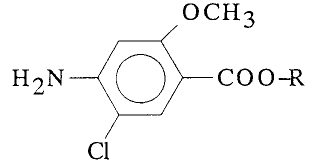
R étant une amine cyclique, généralement un groupement azabicyclo.

Récemment A. Dumuis et al. (*Mol. Pharmacol., 1988,* ***34****, 880-887*) et D.A. Craig et D.E. Clarke (*J. Pharmacol. Exp. Ther., 1990,* ***252****, 1378*-*1396*) ont demontré la présence d'un nouveau type de récepteur de la sérotonine, nommé 5-HT₄, qui stimule la synthèse de l'AMP cyclique dans le système nerveux central (SNC) et dans l'intestin et, presqu'en même temps, A. Dumuis et al. (*Arch. Pharmacol., 1989,* ***340***, *403-410*) ont décrit que certaines amides de l'acide 4-amino-5-chloro-2-méthoxy benzoïque ont une composante agoniste de ce nouveau récepteur 5-HT₄, cet agonisme se traduisant dans l'effet procinétique intestinal de ces produits.

Par contre, K-H. Buckheit et al. (*Eur. J. Pharmacol., 1990,* ***200****, 373-374*) ont décrit récemment un ester de l'acide 4-amino-5-chloro-2-méthoxybenzoïque de formule 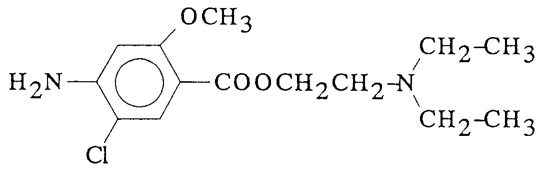
denommé en littérature SDZ-205557, ayant une activité antagoniste du récepteur 5-HT₄.

Encore plus récemment, E. Leung et al. (2nd International Symposium on Serotonin, 15-18 Septembre 1992, Houston USA) ont décrit un autre ester de l'acide 4-amino-5-chloro-2-méthoxybenzoïque de formule 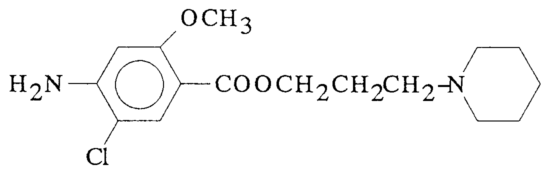
denommé en littérature RS 23597-190, ce composé aussi ayant une activité antagoniste du récepteur 5-HT₄.

Les composés antagonistes du récepteur 5-HT₄ connus actuellement, bien que sélectifs par rapport à la plupart des récepteurs, montrent toujours la présence d'une activité, généralement de type antagoniste, vis-à-vis du récepteur 5-HT₃.

On a maintenant trouvé que le nouveau composé de formule (I) est un antagoniste spécifique du récepteur 5-HT₄, complètement dépourvu d'activité sur les autres récepteurs, y compris le récepteur 5-HT₃.

La présente invention a donc pour object le composé de formule (I) 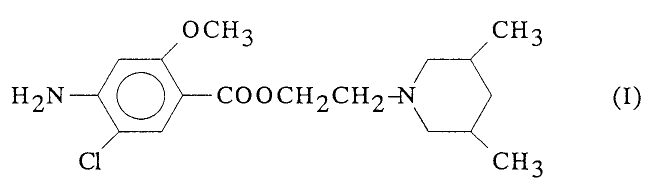
ainsi que ses sels pharmaceutiquement acceptables et ses sels d'ammonium quaternaires.

Les sels pharmaceutiquement acceptables comprennent les sels d'addition avec des acides minéraux ou organiques pharmaceutiquement compatibles, tels que les chlorhydrates, les bromhydrates, les sulfates, les phosphates, les acétates, les tartrates, les succinates, les maléates, les fumarates, les mésylates, les tosylates, etc.

Des sels d'ammonium quaternaires avantageux sont ceux formés à partir de l'azote de la pipéridine, de formule 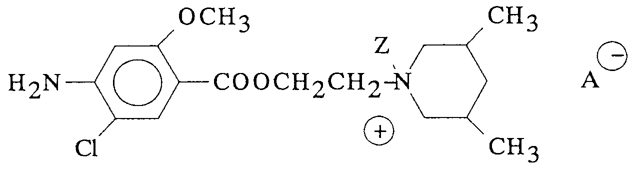
- Z étant un groupe benzyle ou un groupe C₁-C₆ alkyle, de préférence le groupe méthyle, éventuellement substitué, par exemple par un halogène ;
- A étant un anion, tel que le chlorure, le bromure, le iodure, l'acétate, le méthanesulfonate, etc.

La formule (I) ci-dessus comprend soit les isomères purs, cis et trans, selon l'orientation relative des deux groupes méthyliques de la pipéridine, soit le mélange de ces isomères dans n'importe quel rapport.

Les deux isomères cis et trans ont été séparés et leur activité évaluée séparement. On a pu constater que l'isomère cis représente l'isomère le plus actif vis-à-vis du récepteur 5-HT₄.

Le composé de formule (I) ayant la conformation cis, représente donc un composé particulièrement préféré.

La présente invention concerne également un procédé de préparation du composé de formule (I) 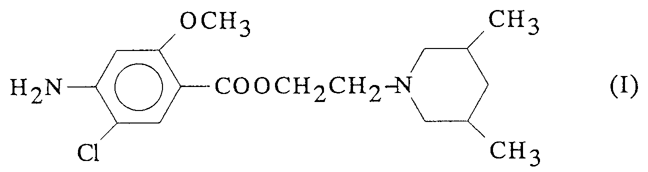
de ses sels pharmaceutiquement acceptables et de ses sels d'ammonium quaternaires, qui prévoit la réaction de l'acide benzoïque de formule (III) 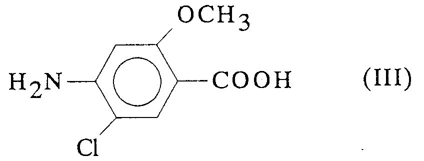
avec du 1,2-dibromoéthane pour obtenir l'intermédiaire de formule (IV) 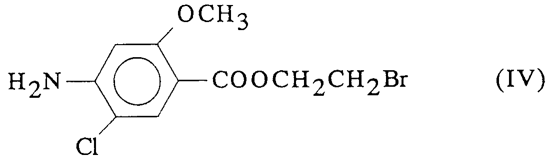
et l'introduction du groupe 3,5-diméthylpipéridino par une simple réaction de substitution nucléophile.

Alternativement, on peut préparer le composé de formule (I) en faisant réagir l'acide benzoïque de formule (V) 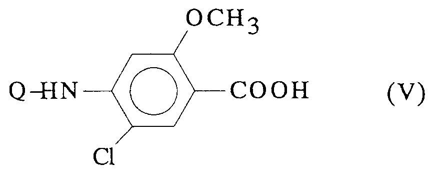
dans laquelle Q représente un groupe protecteur, avec le 3,5-diméthylpipéridinoéthanol pour obtenir l'intermédiaire de formule (VI) 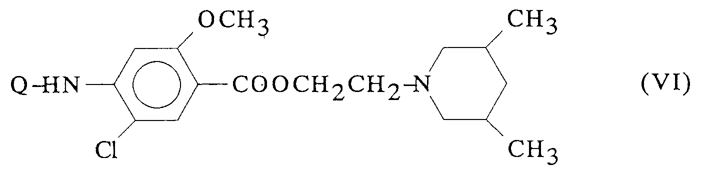
dans laquelle Q est tel que défini ci-dessus, et en éliminant le groupe protecteur Q.

Comme groupes protecteurs on peut employer le groupe trityle, les groupes t-alkoxycarbonyles, tels que le t-butoxycarbonyle (BOC), le t-amyloxycarbonyle (AOC) etc., le groupe trityle étant particulièrement préféré.

L'élimination des groupes protecteurs est aisement effectuée par hydrolyse acide, selon les techniques connues en littérature.

La réaction d'estérification, qui mène au composé intermédiaire de formule (IV), est effectuée selon les méthodes usuelles, de préférence en présence d'une base organique forte comme le 1,8-diazabicyclo[5.4.0]undec-7-ène (DBU).

La réaction de substitution du brome avec la 3,5-diméthylpipéridine est conduite selon les méthodes bien connues, en présence d'une base, comme par exemple les carbonates alcalins, et d'un solvant convenable, comme le DMF.

Le composé (I) ainsi obtenu peut être converti en l'un de ses sels pharmaceutiquement acceptables par réaction avec l'acide approprié dans un solvent convenable, selon les méthodes bien connues.

Lorsque l'on veut préparer les sels d'ammonium quaternaires, on fait réagir le composé de formule (I) avec un excès d'un agent de formule

Z-A

dans laquelle Z et A sont définis comme précédemment, en chauffant le mélange réactionnel à une température comprise entre la température ambiante et le reflux, dans un solvant approprié.

L'invention concerne également l'utilisation du composé de formule (I) ou d'un de ses sels pharmaceutiquement acceptables ou de ses sels d'ammonium quaternaires, pour la préparation de médicaments à action antagoniste du récepteur 5-HT₄ de la sérotonine.

Selon un de ses aspects, l'invention a pour objet l'utilisation du composé de formule (I) ou d'un de ses sels pharmaceutiquement acceptables ou de ses sels d'ammonium quaternaires, en tant que principe actif de médicaments destinés à la prophylaxie et/ou au traitement des pathologies liées au système sérotoninergique lorsque l'on souhaite avoir une action antagoniste sélective du récepteur 5-HT₄.

Dans le but d'évaluer l'activité vis-à-vis du récepteur 5-HT₄, on a soumis les composés de l'invention à l'essai décrit par D.A. Craig et D.E. Clarke. (*J. Pharmacol. Exp. Ther., 1990,* ***252****: 1378-86*) en lui apportant quelques modifications.

Des cobayes albinos mâles - Charles River Italia - (poids moyen de 500 g) sont sacrifiés par décapitation. On isole le plexus myentérique à partir de segments d'iléon de 3 cm environ, prélevés à 10 cm de la jonction iléooeecale ; on place le plexus dans un bain de 20 ml pour organes isolés, contenant une solution de Krebs et de l'acide ascorbique à raison de 1 mg/ml. On maintient la solution, aérée au carboxygène, à la température de 37°C. Le plexus, relié à un transducteur isotonique et soumis à une charge de 0,5 g, est stimulé électriquement de façon supramaximale (fréquence 0,2 Hz, durée 1,5 minutes) par une électrode coaxiale au platine. La préparation est traitée avec de la phénoxybenzamine 10⁻⁷M pendant 30 minutes et, ensuite, lavée plusieurs fois. On réduit le voltage jusqu'à obtenir un effet stimulant de 50% par rapport au maximum et le plexus est ensuite stimulé par la sérotonine à raison de 3·10⁻⁷M. Après lavage et stabilisation, la préparation est prête pour l'essai des composés à activité 5-HT₄. Trois courbes cumulatives de 5-HT sont construites toutes les 60 minutes.
Le composé à examiner est ajouté au bain 30 minutes après la deuxième courbe cumulative de 5-HT. Après 30 minutes d'incubation, on effectue une troisième courbe cumulative de 5-HT (concentration entre 10⁻¹⁰ et 10⁻⁵M). Plusieurs concentrations du produit sont testées et une IC₅₀, dose qui réduit de 50% la réponse de la 5-HT, est calculée.
Dans cet essai, le composé de formule (I) s'est montré très actif, son IC₅₀ étant de l'ordre de quelques nanomoles.
De plus, toujours pour l'évaluation de l'activité 5-HT₄, on a soumis le composé de formule (I) à un essai sur la *tunica muscolaris mucosae* de l'oesophage de rat contracté par du carbacol.
La méthode utilisée est essentiellement celle décrite par G.S. Baxter et D.E. Clarke dans *Eur. J. Pharmacol., 1992,* ***212:*** *225-229.*
Des rats mâles Crl:CD(SD)BR (Charles River Italia) d'un poids moyen de 250 g sont sacrifiés par dislocation cervicale. Un segment d'oesophage d'environ 2 cm est prélevé au niveau intratoraxique à proximité du diaphragme. La *tunica muscolaris mucosae* est ensuite isolée et suspendue dans un bain pour organes isolés de 20 ml contenant une solution de Krebs en présence d'hydrocortisone (3·10⁻⁷ M) à 37°C aérée avec 95% O₂ et 5% CO₂. Le tissu est relié à un transducteur isotonique et soumis à une charge de 1 g. La préparation est incubée avec de la pargiline (10⁻⁴ M) pendant 30 minutes et lavée plusieurs fois. Après 60 minutes environ, on contracte le tissu avec des doses cumulatives de carbacol (10⁻⁶, 2·10⁻⁶, 3·10⁻⁶, 4·10⁻⁶ M); de cette manière, on obtient un tonus du tissu stable dans le temps. La 5-HT (10⁻¹⁰ et 10⁻⁶ M), qui est ajoutée cumulativement, relaxe l'oesophage grâce à l'activation des récepteurs 5-HT₄. Trois courbes de carbacol sont construites par préparation toutes les 90 minutes. Après chaque courbe, on procède au moins à 4 lavages. Aussi bien la contraction provoquée par le carbacol que les effets sur celle-ci sont identiques et peuvent être reproduits.
L'antagoniste (doses simples) est ajouté au bain 30 minutes avant la troisième concentration de carbacol et la courbe cumulative d'inhibition de la 5-HT est construite. On calcule la valeur de pA₂ pour l'antagoniste selon la méthode de O. Arunlakshana et H.O. Schild, *Br. J. Pharmacol., 1959,* ***14***: *48-57*.
Dans cet essai le composé de formule (I) s'est montré très efficace.

Afin de tester l'affinité du composé de formule (I) relativement au récepteur 5-HT₃, on a effectué des essais de liaison in vitro en utilisant les sites de liaison 5-HT₃ présents dans le cortex cérébral du rat (G.J. Kilpatrick, B.J. Jones et M.B. Tyers, *Nature*, *1987;* ***330****: 746-8*) et comme ligand marqué le [³H] BRL 43694 (granisetron), un antagoniste des récepteurs 5-HT₃ puissant et spécifique, à la concentration de 0,5 nM.

La préparation des membranes et le test de liaison ont été effectués selon la méthode décrite par Nelson et Thomas (D.R. Nelson et D.R. Thomas, *Biochem. Pharmacol., 1989;* ***38****: 1693-5*).

Les résultats ont été évalués avec des méthodes d'ajustement ("fitting") non linéaire "Accufit saturation", pour les études de saturation (H.A. Feldman, *Analyt*. *Biochem., 1972;* ***48***: *317-38*) et "Accufit competition", pour les études de déplacement (H.A. Feldman, D. Rodbard et D. Levine, *Analyt. Biochem., 1972;* ***45****: 530-56*).

Le composé de formule (I) s'est montré inactif dans le déplacement du [³H] BRL 43694.

Compte-tenu des résultats des essais sus-mentionnés, le composé de formule (I), ainsi que ses sels pharmaceutiquement acceptables et ses sels d'ammonium quaternaires, peuvent être utilisés dans la préparation de médicaments destinés au traitement et/ou à la prophylaxie des troubles du système sérotoninergique, soit périphérique soit central, qui impliquent les récepteurs 5-HT₄, lorsqu'une action antagoniste sélective est demandée.

Lesdits médicaments peuvent être employés dans le traitement et/ou la prophylaxie des affections du système gastroentérique, du système cardiovasculaire ainsi que du système nerveux central.

Plus particulièrement, ces médicaments peuvent être indiqués par exemple dans les troubles de la fonction intestinale, tels que la syndrôme du colon irritable, l'excessive sécrétion gastrointestinale, I'ulcère peptique, la dyspepsie, la nausée, le vomissement, la diarrhée de différentes origines, dans les troubles de la cognition, tels que la démence sénile, la maladie d'Alzheimer, la psychose, la dépression, l'anxiété, ainsi que dans les diskinésies, dans la dépendance d'alcool ou de drogues, comme analgésique, par exemple dans les migraines, dans les troubles cardiovasculaires, comme les arythmies et dans les troubles respiratoires.

La présente invention a également pour objet le composé de formule (I) ainsi que ses sels pharmaceutiquement acceptables et ses sels d'ammonium quaternaires marqués sur l'un de ses atomes par des isotopes.

Grâce à son activité puissante et sélective vis-à-vis du récepteur 5-HT₄, le composé de formule (I), lorsqu'il est marqué, peut être utilisé en tant qu'outil dans les études sur ce récepteur, en particulier dans les essais biochimiques.

La présente invention concerne également des compositions pharmaceutiques renfermant, en tant que principe actif, le composé de formule (I) ou un de ses sels d'addition d'acide ou de ses sels d'ammonium quaternaires, pharmaceutiquement acceptables, purs ou en association avec toute autre substance pharmacologiquement compatible, destinées à l'administration par voie orale et/ou parentérale.

Pour l'administration orale on peut préparer des comprimés, des poudres, des granules, des pilules, des dragées, des sirops, des solutions, des émulsions, des suspensions, des formes gastro-résistantes.

Pour l'administration parentérale, on peut préparer des compositions stériles injectables, aqueuses ou non-aqueuses, des suppositoires pour l'administration rectale, des patches pour l'administration transdermique, des formulations aptes à l'administration oculaire et nasale.

On peut également préparer des formes pharmaceutiques particulières comme les formes lyposomiques, les formes retard, les formes dans lesquelles le principe actif est inclus dans des cyclodextrines.

Les formes pharmaceutiques sont préparées selon les méthodes usuelles en mélangeant le principe actif aux excipients convenables, tels que l'amidon, le stéarate de magnésium, le talc, le lactose, le saccharose, l'huile de paraffine, les produits mouillants, aromatisants, stabilisants, etc.

Le dosage approprié du principe actif doit être évalué selon la voie d'administration, les caractéristiques du sujet à traiter, tels que l'âge, le poids du corps et la gravité des affections à traiter; généralement, le dosage est compris entre 0,05 et 20 mg/kg, notamment entre 0,1 et 10 mg/kg, de préférence entre 1 et 5 mg/kg.

Les exemples qui suivent illustrent mieux l'invention sans toutefois la limiter.

### EXEMPLE 1

**Préparation de l'ester 3,5-diméthylpipéridinoéthylique de l'acide 4-amino-5-chloro-2-méthoxybenzoïque (I).**

### a) Ester 2-bromoéthylique de l'acide 4-amino-5-chloro-2-méthoxybenzoïque.

A un mélange de 20,1 g (0,1 mol) d'acide 4-amino-5-chloro-2-méthoxybenzoïque, 100 ml de 1,2-dibromoéthane et 200 ml de THF sont ajoutés 15,2 g (0,1 mol) de 1,8-diazabicyclo[5.4.0]undec-7-ène au reflux. Le mélange réactionnel est maintenu au reflux pendant 3 heures. Après avoir refroidi, les précipités sont filtrés et le solvant est évaporé sous vide. Le résidu est repris par du CH₂Cl₂, lavé avec une solution saturée de NaCl et séché sur MgSO₄. La solution est filtrée sur silice. Après évaporation du solvant, on obtient 22 g (Rdt: 71%) d'ester. Recristallisation: éther/pentane.
¹H 200 MHz (CDCl3) δ (ppm) : 7,80 (s, 1H Arom); 6,22 (s, 1H Arom); 4,46 (t, J=6 Hz, -OCH₂-); 4,45 (s, -NH₂); 3,78 (s, -OCH₃); 3,54 (t, J=6 Hz, -CH₂Br).

### b) Ester 3,5-diméthylpipéridinoéthylique de l'acide 4-amino-5-chloro-2-méthoxybenzoïque.

Un mélange de 1,54 g (5 mmol) du composé de l'étape a), 0,69 g (5 mmol) de K₂CO₃ et 1,13 g (10 mmol) de 3,5-diméthylpipéridine dans 30 ml de DMF est agité à 50°C pendant 3 heures. Le mélange réactionnel est filtré, le filtrat est évaporé et repris au CH₂Cl₂, lavé avec une solution saturée de NaCl, séché sur MgSO₄ et évaporé pour donner 1,3 g (76%) du composé (I). Recristallisation: AcOEt/hexane. P.f.: 115°C.

Le composé de formule (I), tel qu'obtenu par les réactions de l'Exemple 1, examiné en chromatographie sur couche mince (éluant = acétate d'éthyle), montre deux taches, qui correspondent aux deux isomères cis et trans.

Les deux isomères ont été séparés par chromatographie sur colonne et l'attribution de la stéréochimie cis et trans des deux isomères a été effectuée par l'examen de leurs spectres RMN (¹H et ¹³C).

On a ainsi vérifié que le composé, qui se sépare plus rapidement par chromatographie en éluant à l'acétate d'éthyle, correspond à l'isomère trans, tandis que celui plus polaire correspond à l'isomère cis.

Voici les données les plus significatives pour la détermination des conformations 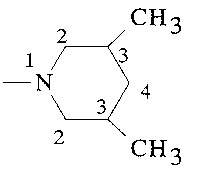
- Isomère trans;: spectre ¹H-RMN (CDCl₃): ppm 0,96 (d, 6H, CH₃-C₍₃₎); 1,28 (m, 2H, H₂C₍₄₎); 1,90 (m, 2H, HC₍₃₎); 2,14 (m, 2H, H₂C₍₂₎); 2,50 (m, 2H, H₂C₍₂₎).
spectre ¹³C-RMN (CDCl₃): ppm 19,0 (CH₃-C₍₃₎); 27,3 (C₍₃₎); 38,8 (C₍₄₎).
- Isomère cis;: spectre ¹H-RMN (CDCl₃): ppm 0,51 (m, 1H, Hₐₓ-C₄); 0,87 (d, 6H, CH₃C₍₃₎); 1,66 (m, 5H, Hₐₓ-C₍₂₎, HC₍₃₎, Hₐₓ-C₍₂₎); 2,92 (m, 2H, H_{eq.}-C₍₂₎).
spectre ¹³C-RMN (CDCl₃): ppm 19,5 (CH₃-C); 31,0 (C₍₃₎); 31,9 (C₍₄₎).

### EXEMPLE 2

**Iodure de 1-[2-(4-amino-5-chloro-2-méthoxybenzoyloxy)éthyl]-1,3,5-triméthylpipéridinium - Isomère cis**

On mélange 0,50 g de l'isomère cis de l'ester 3,5-diméthylpipéridinoéthylique de l'acide 4-amino-5-chloro-2-méthoxybenzoïque et 0,20 ml de iodure de méthyle dans 20 ml d'acétonitrile et on porte à reflux pendant 3 heures. Après l'évaporation du solvant, le résidu est recristallisé dans l'éthanol pour donner 0,50 g du composé du titre. P.f. 223°C.

### EXEMPLE 3

**Iodure de 1-[2-(4-amino-5-chloro-2-méthoxybenzoyloxy)éthyl]-1,3,5-triméthylpipéridinium - Isomère trans**

On suit la méthode de l'Exemple 2 en utilisant 0,20 g de l'isomère trans de l'ester 3,5-diméthylpipéridinoéthylique de l'acide 4-amino-5-chloro-2-méthoxybenzoïque au lieu de l'isomère cis. On obtient 0,23 g du composé du titre. P.f. 223°C.

## Revendications

1. Composé de formule (I) 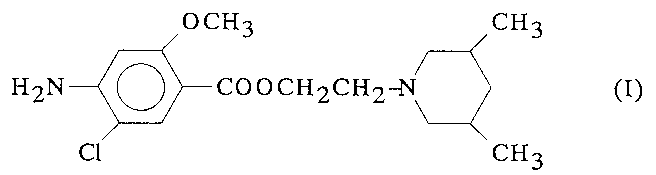 ainsi que ses sels pharmaceutiquement acceptables et ses sels d'ammonium quaternaires.

2. Composé selon la revendication 1, sous forme de chlorhydrate.

3. Composé selon la revendication 1, caractérisé en ce que la conformation des deux groupes méthyle de la pipéridine est cis.

4. Composé selon la revendication 1, caractérisé en ce que la conformation des deux groupes méthyle de la pipéridine est trans.

5. Composition pharmaceutique contenant en tant que principe actif le composé de formule (I) 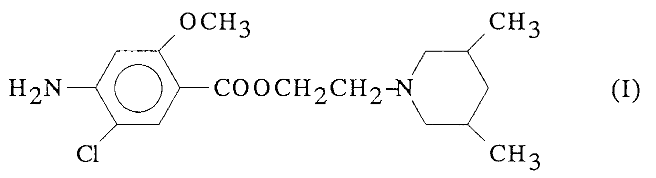 ou un de ses sels d'addition d'acide ou de ses sels d'ammonium quaternaires, pharmaceutiquement acceptables.

6. Composition pharmaceutique selon la revendication 5, caractérisée en ce qu'elle contient en tant que principe actif le composé de la revendication 3.

7. Composition pharmaceutique selon la revendication 5, caractérisée en ce qu'elle contient en tant que principe actif le composé de la revendication 4.

8. Utilisation d'un composé selon la revendication 1 pour la préparation d'un médicament à activité antagoniste sélective du récepteur 5-HT₄.

9. Utilisation selon la revendication 8 pour la préparation d'un médicament destiné au traitement et/ou à la prophylaxie des troubles gastroentériques, des troubles cardiaques, des troubles du système nerveux central, et des troubles respiratoires.

10. Procédé pour la préparation du composé de formule (I) 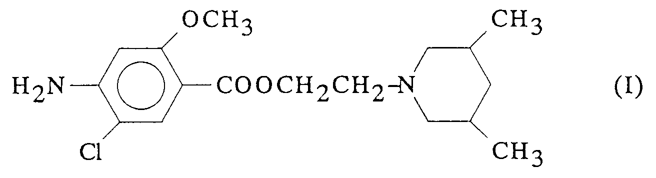 de ses sels pharmaceutiquement acceptables, de ses sels d'ammonium quaternaires et de ses isomères, caractérisé en ce que
- soit on fait réagir l'acide benzoïque de formule (III) 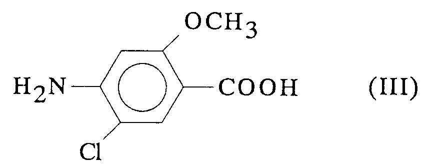 avec du 1,2-dibromoéthane pour obtenir l'intermédiaire de formule (IV) 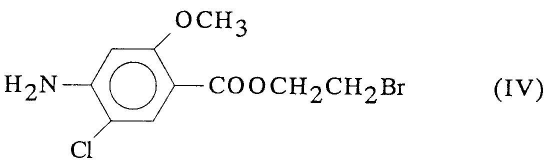 et on fait réagir l'intermédiaire de formule (IV) avec la 3,5-diméthylpipéridine,
- soit on fait réagir l'acide benzoïque de formule (V) 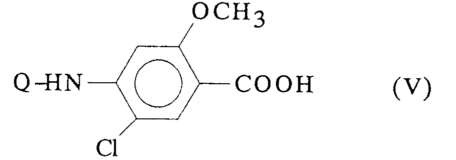 dans laquelle Q représente un groupe protecteur, avec le 3,5-diméthylpipéridinoéthanol pour obtenir l'intermédiaire de formule (VI) 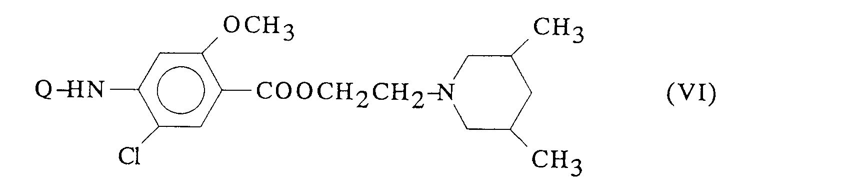 dans laquelle Q est tel que défini ci-dessus, et on élimine le groupe protecteur Q; éventuellement on sépare par chromatographie sur colonne les isomères du composé de formule (I) ainsi obtenu et éventuellement on transforme le composé (I) ainsi obtenu en l'un de ses sels pharmaceutiquement acceptables ou en l'un de ses sels d'ammonium quaternaires.
